# FASCICULE DE BREVET EUROPEEN

(11) **EP 3 322 352 B1**
(45) Date de publication et mention de la délivrance du brevet: **04.11.2020**
(21) Numéro de dépôt: 16750946.2
(22) Date de dépôt: 11.07.2016
(51) Int. Cl.: A61B 17/06, A61C 3/04, A61F 17/00, A61C 19/02, A61B 17/122, A61M 25/00, A61B 50/30, A61B 50/31, A61B 50/22, A61M 5/00

(54) **ENSEMBLE D'INSTRUMENTATION CHIRURGICALE**
CHIRURGISCHE INSTRUMENTENANORDNUNG
SURGICAL INSTRUMENTATION ASSEMBLY

(30) Priorité: 16.07.2015 FR 1556718
(43) Date de publication de la demande: 23.05.2018
(73) Titulaire: NovaStep, 35760 Saint-Grégoire (FR)
(72) Inventeur: GIROD, Loïc, 35580 Goven (FR); LE BESQUE, Rémi, 35170 Bruz (FR); ZELENSKY, Nicholas E., New York, NY 12472 (US)
(74) Mandataire: Chevalier, Renaud Philippe
(86) Numéro de dépôt international: PCT/FR2016/051781
(87) Numéro de publication internationale: WO 2017/009571

(56) Documents cités:
- FR-A- 912 281
- FR-A1- 2 580 490
- GB-A- 2 396 550
- US-A- 3 058 579
- US-A- 4 327 060
- US-A- 4 856 648

## Description

La présente invention concerne un ensemble d'instrumentation chirurgicale comprenant plusieurs instruments chirurgicaux.

La présente invention s'applique au domaine de la chirurgie, pour toute opération chirurgicale nécessitant de mettre des instruments chirurgicaux à disposition de l'équipe chirurgicale.

L'état de la technique comprend par exemple une boîte dans laquelle de nombreux instruments chirurgicaux sont emballés, parfois individuellement. Une telle boîte a de grandes dimensions, car elle est polyvalente, afin de contenir tous les instruments chirurgicaux possiblement nécessaires pour plusieurs types d'opérations. GB 2 396 550 A divulgue un ensemble d'instrumentation chirurgicale comprenant un tube de stockage, plusieurs instruments chirurgicaux et un support comportant plusieurs éléments de fixation.

Cependant, les ensembles d'instrumentation chirurgicale de l'état de la technique sont encombrants. En outre, un ensemble d'instrumentation chirurgicale de l'état de la technique est relativement difficile à manipuler, ce qui allonge la durée de l'opération chirurgicale.

La présente invention a notamment pour but de résoudre, en tout ou partie, les problèmes mentionnés ci-avant. Dans ce but, la présente invention a pour objet un ensemble d'instrumentation chirurgicale comprenant au moins :
- un tube de stockage présentant une ouverture de service,
- un organe d'obturation configuré pour obturer l'ouverture de service,
- plusieurs instruments chirurgicaux destinés à un usage temporaire, et
- un support comportant plusieurs éléments de fixation configurés pour fixer les instruments chirurgicaux au support de manière détachable, le support et les instruments chirurgicaux formant un sous-ensemble lorsque les instruments chirurgicaux sont fixés au support ;
le tube de stockage étant configuré pour loger le sous-ensemble, de façon à stocker le sous-ensemble à l'état stérile lorsque l'organe d'obturation obture l'ouverture de service, et le sous-ensemble étant conformé pour être extrait du tube de stockage à travers l'ouverture de service et les caractéristiques supplémentaires mentionnées dans la revendication 1.

Ainsi, un tel ensemble d'instrumentation chirurgicale permet de stocker plusieurs instruments chirurgicaux stériles de manière compacte, tout en permettant une utilisation rapide en salle d'opération, car un opérateur peut sortir en une seule action tous les instruments chirurgicaux nécessaires.

Dans la présente demande, le terme « tube de stockage » désigne un corps creux allongé, c'est-à-dire plus long que large. Par exemple, ce corps creux peut avoir une forme globalement cylindrique ou prismatique.

Dans la présente demande, le terme « instrument chirurgical » peut désigner i) un instrument chirurgical complet, qu'un chirurgien peut manipuler de manière autonome, ou ii) une partie d'un instrument chirurgical, qu'un chirurgien peut assembler à une autre partie avant manipulation.

Selon une variante, le tube de stockage est configuré pour loger totalement le sous-ensemble.

Alternativement à cette variante, le tube de stockage est configuré pour loger la plus grande partie du sous-ensemble, et l'organe d'obturation est configuré pour loger le reste du sous-ensemble. Dans cette alternative, une petite partie du sous-ensemble dépasse du tube de stockage mais est couverte par l'organe d'obturation. Cette alternative permet de former un ensemble d'instrumentation chirurgicale particulièrement compact et elle facilite la préhension du sous-ensemble.

Selon un mode de réalisation, les instruments chirurgicaux sont sélectionnés dans le groupe constitué d'embouts de tournevis, de forets, de broches, d'enveloppes tubulaires contenant au moins une broche, de fraises à chambrer, de jauges et de réglets de mesure.

Ainsi, de tels instruments chirurgicaux permettent de réaliser la plupart des gestes nécessaires au cours d'une opération de chirurgie orthopédique.

Selon une variante, le nombre d'instruments chirurgicaux est compris entre 2 et 15.

Selon un mode de réalisation, l'ouverture de service est située à une extrémité du tube de stockage.

Comme le tube de stockage est un corps creux allongé selon une direction longitudinale, le tube de stockage a deux extrémités opposées selon la direction longitudinale.

Alternativement à ce mode de réalisation, l'ouverture de service peut être située sur une paroi latérale du tube de stockage, cette paroi latérale s'étendant entre les extrémités opposées du tube de stockage.

Selon une variante, l'organe d'obturation est lié au tube de stockage de manière détachable.

Alternativement à cette variante, l'organe d'obturation peut être articulé au tube de stockage de façon à passer d'une position d'obturation, dans laquelle l'organe d'obturation obture l'ouverture de service, à une position d'ouverture, dans laquelle l'organe d'obturation dégage l'ouverture de service, l'organe d'obturation restant lié au tube de stockage. Ainsi, l'organe d'obturation ne peut pas être perdu dans la salle d'opération.

Selon l'invention, le support comprend un manche adapté pour être manipulé par un opérateur, le manche ayant une portion d'attache configurée pour attacher sélectivement l'un des instruments chirurgicaux de manière détachable.

Ainsi, un tel manche permet de définir un ensemble d'instrumentation chirurgicale compact et autonome, car le manche permet au chirurgien de manipuler les instruments chirurgicaux.

Selon une variante, la portion de verrouillage est configurée pour un verrouillage d'un instrument chirurgical.

Selon l'invention, des éléments de fixation sont formés par des rainures longitudinales formées à la surface externe du manche, chaque rainure longitudinale étant adaptée pour loger une partie d'un instrument chirurgical respectif de façon à fixer ledit instrument chirurgical par encliquetage élastique.

Ainsi, de telles rainures longitudinales permettent de former un manche compact avec les instruments chirurgicaux.

Selon une variante, les éléments de fixation comprennent en outre un ruban agencé autour du manche de façon à retenir des instruments chirurgicaux.

Selon une variante, les éléments de fixation comprennent en outre un élément de bridage thermorétractable.

Selon un mode de réalisation, le manche présente une cavité centrale adaptée pour loger au moins un instrument chirurgical.

Ainsi, cette cavité centrale rend l'ensemble d'instrumentation particulièrement compact.

Selon un mode de réalisation, le tube de stockage a au moins une partie translucide, par exemple une partie transparente.

Ainsi, un tel tube de stockage permet de vérifier la présence de tous les instruments chirurgicaux nécessaires à une opération chirurgicale, avant d'ouvrir le tube de stockage stérliisé. Selon une variante, le tube de stockage est totalement translucide, par exemple totalement transparent.

Selon un mode de réalisation, le tube de stockage (2) est composé d'un matériau sélectionné dans le groupe constitué d'un polyéthylène téréphtalate glycol (PETG), d'un polycarbonate (PC) et d'un polypropylène.

Ainsi, un tel matériau permet de fabriquer un tube de stockage résistant aux chocs et vibrations, ainsi qu'au procédé de stérilisation, par exemple une irradiation par rayons gamma.

Selon un mode de réalisation, le tube de stockage a une longueur comprise entre 70 mm et 300 mm, et dans lequel le tube de stockage a une dimension transversale, mesurée dans un plan perpendiculaire à la longueur, comprise entre 20 mm et 100 mm.

Par exemple, dans la variante où le tube de stockage est cylindrique à base circulaire, le tube de stockage peut avoir un diamètre compris entre 20 mm et 100 mm.

Selon une variante, un rapport entre :
i) au numérateur : l'encombrement du sous-ensemble,
ii) au dénominateur : le volume interne du tube de stockage,
est compris entre 0,05% et 95%.

Ainsi, un tel tube de stockage est particulièrement compact.

Selon un mode de réalisation, l'ensemble d'instrumentation chirurgicale selon l'invention, comprenant en outre :
- un tube de protection configuré pour loger le tube de stockage, et
- un bouchon externe configuré pour boucher le tube de protection lorsque le tube de protection loge le tube de stockage.

Ainsi, un tel tube de protection permet de protéger le tube de stockage, donc de protéger les instruments chirurgicaux contre des chocs de forte intensité.

Les modes de réalisation et les variantes mentionnés ci-avant peuvent être pris isolément ou selon toute combinaison techniquement possible.

La présente invention sera bien comprise et ses avantages ressortiront aussi à la lumière de la description qui va suivre, donnée uniquement à titre d'exemple non limitatif et faite en référence aux figures schématiques annexées, dans lesquelles des signes de références identiques correspondent à des éléments structurellement et/ou fonctionnellement identiques ou similaires. Dans les figures schématiques annexées :
- la figure 1 est une vue en perspective d'un ensemble d'instruments chirurgicaux selon un premier mode de réalisation, à l'état ouvert en vue d'une opération chirurgicale ;
- la figure 2 est une vue en perspective d'un sous-ensemble appartenant à l'ensemble d'instruments chirurgicaux de la figure 1, à l'état enroulé ;
- la figure 3 est une vue en perspective du sous-ensemble de la figure 2, à l'état déroulé ;
- la figure 4 est une vue en perspective d'un ensemble d'instruments chirurgicaux selon un deuxième mode de réalisation, à l'état fermé et stérile ;
- la figure 5 est une vue en perspective d'un sous-ensemble appartenant à l'ensemble d'instruments chirurgicaux de la figure 4, le sous-ensemble comportant plusieurs instruments chirurgicaux ;
- la figure 6 est une vue en perspective du sous-ensemble de la figure 5 dépourvu des instruments chirurgicaux ;
- la figure 7 est une vue du dessus du sous-ensemble de la figure 5 ;
- la figure 8 est une vue de face d'un ensemble d'instruments chirurgicaux selon un mode de réalisation de l'invention, à l'état fermé et stérile ;
- la figure 9 est une vue de face de l'ensemble d'instruments chirurgicaux de la figure 8, à l'état semi-ouvert ;
- la figure 10 est une vue en perspective de l'ensemble d'instruments chirurgicaux de la figure 8, à l'état ouvert ;
- la figure 11 est une vue en perspective, dans un premier état, d'un sous-ensemble appartenant à l'ensemble d'instruments chirurgicaux de la figure 8, le sous-ensemble comportant plusieurs instruments chirurgicaux ; et
- la figure 12 est une vue en perspective, dans un deuxième état, du sous-ensemble de la figure 11, le sous-ensemble comportant plusieurs instruments chirurgicaux.

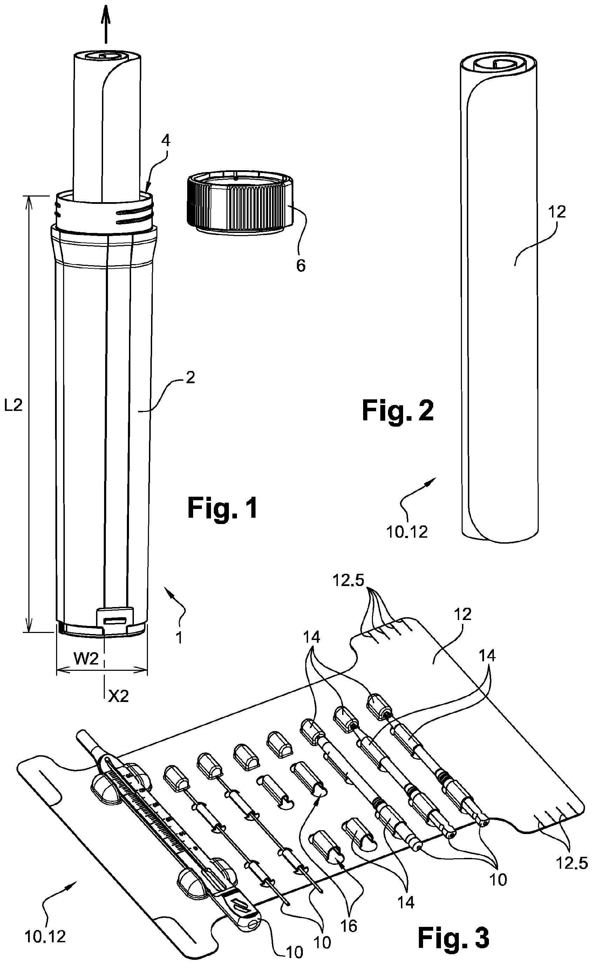

Les figures 1, 2 et 3 illustrent un ensemble d'instrumentation chirurgicale 1 comprenant un tube de stockage 2 présentant une ouverture de service 4 et un organe d'obturation 6 configuré pour obturer l'ouverture de service 4.

De plus, l'ensemble d'instrumentation chirurgicale 1 plusieurs instruments chirurgicaux 10 destinés à un usage temporaire et un support 12 comportant plusieurs éléments de fixation 14. Les éléments de fixation 14 sont configurés pour fixer les instruments chirurgicaux 10 au support 12 de manière détachable. Dans l'exemple des figures 1 à 3, chaque élément de fixation 14 est configuré pour fixer un instrument chirurgical respectif 10 au support 12 de manière détachable.

Le support 12 et les instruments chirurgicaux 10 forment un sous-ensemble 10.12 lorsque les instruments chirurgicaux 10 sont fixés au support 12, comme l'illustre la figure 3. Le sous-ensemble 10.12 comprend donc le support 12 et les instruments chirurgicaux 10. Les instruments chirurgicaux 10 comprennent ici des embouts de tournevis, des forets, des broches, une jauge et un réglet de mesure.

Le tube de stockage 2 est configuré pour loger totalement le sous-ensemble 10.12, de façon à stocker le sous-ensemble 10.12 à l'état stérile lorsque l'organe d'obturation 6 obture l'ouverture de service 4. Le tube de stockage 2 est un corps creux allongé selon une direction longitudinale X2. Le tube de stockage 2 a deux extrémités opposées selon la direction longitudinale X2.

L'ouverture de service 4 est ici située à une extrémité du tube de stockage 2. Comme l'illustre la figure 1, le sous-ensemble 10.12 est conformé pour être extrait du tube de stockage 2 à travers l'ouverture de service 4.

Le support 12 est totalement flexible de façon à passer :
- d'une configuration de stockage (figures 1 et 2), dans laquelle le support 12 enveloppe les instruments chirurgicaux 10 de sorte que le sous-ensemble 10.12 peut être logé dans le tube de stockage 2,
- à une configuration de service (figure 3), dans laquelle le support 12 est déployé de sorte qu'un opérateur peut détacher les instruments chirurgicaux 10 des éléments de fixation 14.

Dans l'exemple des figures 1 à 3, le support 12 est formé par un film composé de matériau plastique. Le support 12 est à l'état enroulé en configuration de stockage (figures 1 et 2) et à l'état déroulé en configuration de service (figure 3). Le support 12 présente des fentes prédécoupées 12.5 qui ont pour fonction de maintenir le film à l'état enroulé. À cet effet, chaque fente prédécoupée peut recevoir une partie du film.

Dans l'exemple des figures 1 à 3, les éléments de fixation 14 forment des passages 16. Chaque passage 16 est configuré pour passer au moins une partie d'un instrument chirurgical respectif 10. Un passage 16 peut être formé en découpant deux entailles parallèles de part et d'autre d'un pont de matières, de sorte que ce pont de matière peut retenir un instrument chirurgical 10.

L'ensemble d'instrumentation chirurgicale 1 permet de stocker plusieurs instruments chirurgicaux 10 de manière compacte et à l'état stérile, tout en permettant une utilisation rapide en salle d'opération, car un opérateur peut sortir en une seule action tous les instruments chirurgicaux 10 nécessaires. En l'occurrence, le support 12 permet de retenir huit instruments chirurgicaux 10.

Pour fixer un instrument chirurgical 10 sur le support 12, un opérateur peut enfiler l'instrument chirurgical 10 sous le pont de matière. Pour détacher l'instrument chirurgical 10 du support 12, il suffit à l'opérateur de tirer l'instrument chirurgical 10 pour le glisser sous le pont de matière jusqu'à libérer l'instrument chirurgical 10, ce qui est rapide.

Par ailleurs, le tube de stockage 2 est totalement transparent, ce qui permet de vérifier, avant d'ouvrir le tube de stockage 2, la présence de tous les instruments chirurgicaux 10 nécessaires à une opération chirurgicale. Le tube de stockage 2 peut être composé de polyéthylène téréphtalate glycol (PETG).

Le tube de stockage 2 est ici cylindrique à base circulaire avec un diamètre compris environ égal à 30 mm. Le tube de stockage 2 a ici une longueur L2 environ égale à 150 mm et une dimension transversale W2 (diamètre), mesurée dans un plan perpendiculaire à la longueur L2, environ égale à 30 mm.

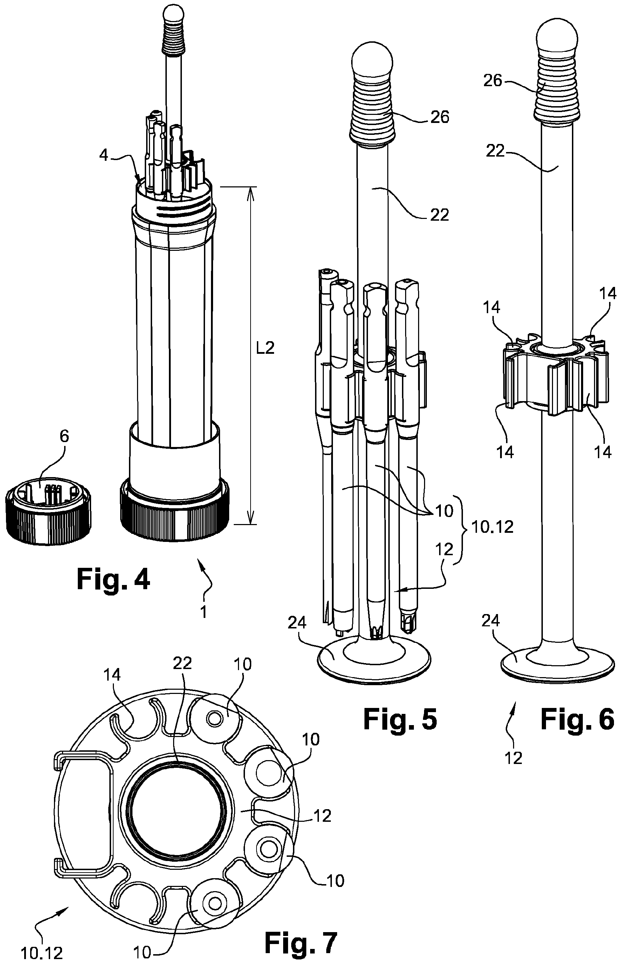

Les figures 4, 5 ,6 et 7 illustrent un ensemble d'instrumentation chirurgicale 1 conforme à un deuxième mode de réalisation. Dans la mesure où l'ensemble d'instrumentation chirurgicale 1 des figures 4 à 7 est similaire à l'ensemble d'instrumentation chirurgicale 1 des figures 1 à 3, la description de l'ensemble d'instrumentation chirurgicale 1 donnée ci-avant en relation avec les figures 1 à 3 peut être transposée à l'ensemble d'instrumentation chirurgicale 1 des figures 4 à 7, à l'exception des différences notables énoncées ci-après.

L'ensemble d'instrumentation chirurgicale 1 des figures 4 à 7 diffère de l'ensemble d'instrumentation chirurgicale 1 des figures 1 à 3, notamment car le support 12 comprend une tige 22 à laquelle sont solidarisés les éléments de fixation 14, alors que le support 12 des figures 1 à 3 est un film flexible. Les éléments de fixation 14 sont distribués sur le pourtour de la tige 22, comme le montre la figure 6.

L'ensemble d'instrumentation chirurgicale 1 des figures 4 à 7 diffère de l'ensemble d'instrumentation chirurgicale 1 des figures 1 à 3, notamment car chaque élément de fixation 14 est ici formé par un collier qui est élastiquement déformable de façon à fixer des instruments chirurgicaux 10 par encliquetage élastique. Les éléments de fixation 14 sont distribués sur tout le pourtour de la tige 22.

La tige 22 est sensiblement parallèle à une direction longitudinale du tube de stockage 2 lorsque le sous-ensemble 10.12 est logé dans le tube de stockage 2, comme l'illustre la figure 5.

En l'occurrence, la tige 22 et les éléments de fixation 14 permettent de retenir sept instruments chirurgicaux 10. Un opérateur peut tirer un instrument chirurgical 10 loin de la tige 22 pour détacher cet instrument chirurgical 10. La tige 22 forme un support 12 très peu encombrant et permettant d'accéder facilement à tous les instruments chirurgicaux 10.

Par ailleurs, la tige 22 a une portion de préhension 26 qui est située dans une région d'extrémité de la tige 22. La portion de préhension 26 est située près de l'ouverture de service 4 lorsque le tube de stockage 2 loge le sous-ensemble 10.12.

La portion de préhension 26 permet à un opérateur de prendre le sous-ensemble 10.12, puis de l'extraire hors du tube de stockage 2. Ensuite, la portion de préhension 26 permet de tenir la tige 22 pour détacher un instrument chirurgical 10 du support 12.

Le support 12 a un socle 24 disposé à une région d'extrémité de la tige 22. En l'occurrence, le socle 24 est situé à l'opposé de la portion de préhension 26. Le socle 24 est configuré pour reposer sur une surface horizontale dans la salle d'opérations. Le socle 24 est situé dans la région d'extrémité opposée à la portion de préhension 26.

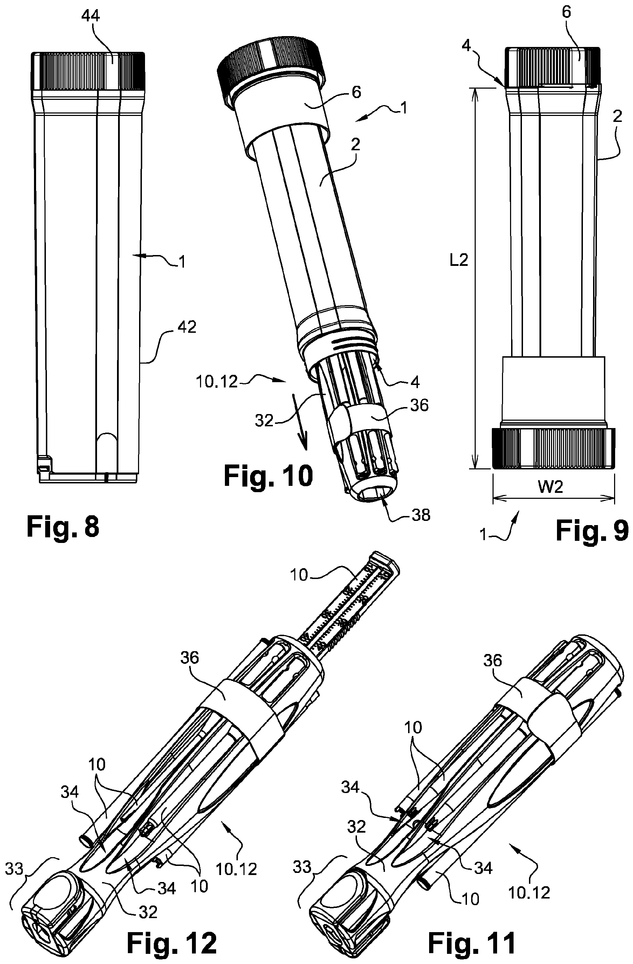

Par ailleurs, l'ensemble d'instrumentation chirurgicale 1 des figures 4 à 7 comprend aussi un tube de protection non représenté, qui est similaire, structurellement et fonctionnellement, au tube de protection 42 illustré à la figure 8.

Les figures 8, 9, 10, 11 et 12 illustrent un ensemble d'instrumentation chirurgicale 1 conforme à un mode de réalisation de l'invention. Dans la mesure où l'ensemble d'instrumentation chirurgicale 1 des figures 8 à 12 est similaire à l'ensemble d'instrumentation chirurgicale 1 des figures 4 à 7, la description de l'ensemble d'instrumentation chirurgicale 1 donnée ci-avant en relation avec les figures 4 à 7 peut être transposée à l'ensemble d'instrumentation chirurgicale 1 des figures 8 à 12, à l'exception des différences notables énoncées ci-après.

L'ensemble d'instrumentation chirurgicale 1 des figures 8 à 12 diffère de l'ensemble d'instrumentation chirurgicale 1 des figures 4 à 7, notamment car le support 12 comprend un manche 32 adapté pour être manipulé par un opérateur, alors que l'ensemble d'instrumentation chirurgicale 1 des figures 4 à 7 comprend la tige 22. Le manche 32 a une portion de verrouillage 33 qui est configurée pour attacher ou détacher sélectivement (au manche 32) l'un des instruments chirurgicaux 10 de manière détachable. La portion d'attache 33 est configurée pour permettre un verrouillage rapide d'un instrument chirurgical 10.

De plus, l'ensemble d'instrumentation chirurgicale 1 des figures 8 à 12 diffère de l'ensemble d'instrumentation chirurgicale 1 des figures 4 à 7, notamment car la plupart des éléments de fixation 14 sont formés par des rainures longitudinales 34 formées à la surface externe du manche 32, alors que la tige 22 comporte des colliers pour fixer les instruments chirurgicaux 10.

Chaque rainure longitudinale 34 est adaptée pour loger une partie d'un instrument chirurgical respectif 10 de façon à fixer cet instrument chirurgical 10 par encliquetage élastique. En l'occurrence, le manche 32 permet de retenir huit instruments chirurgicaux 10.

Comme l'illustrent les figures 11 et 12, les éléments de fixation 14 comprennent en outre un ruban 36 qui est agencé autour du manche 32 de façon à retenir des instruments chirurgicaux 10.

En outre, l'ensemble d'instrumentation chirurgicale 1 des figures 8 à 12 diffère de l'ensemble d'instrumentation chirurgicale 1 des figures 4 à 7, notamment car le manche 32 présente une cavité centrale 38 qui est adaptée pour loger au moins un instrument chirurgical 10, en l'occurrence un réglet, alors que la tige 22 est pleine.

L'ensemble d'instrumentation chirurgicale 1 comprend en outre un tube de protection 42 qui est configuré pour loger le tube de stockage 2. De plus, l'ensemble d'instrumentation chirurgicale 1 comprend un bouchon externe 44 configuré pour boucher le tube de protection 42 lorsque le tube de protection 42 loge le tube de stockage 2.

Le tube de protection 42 et le bouchon externe 44 sont seulement visibles à la figure 8 illustrant le troisième mode de réalisation. Cependant, les premier et deuxième modes de réalisation peuvent également comprendre un tube de protection et un bouchon externe non représentés.

Bien entendu, la présente invention n'est pas limitée aux modes de réalisation particuliers décrits dans la présente demande de brevet, ni à des modes de réalisation à la portée de l'homme du métier. D'autres modes de réalisation peuvent être envisagés sans sortir du cadre de l'invention, à partir de tout élément équivalent à un élément indiqué dans la présente demande de brevet. L'invention est définie par les revendications suivantes.

## Revendications

1. Ensemble d'instrumentation chirurgicale (1) comprenant au moins :
- un tube de stockage (2) présentant une ouverture de service (4),
- un organe d'obturation (6) configuré pour obturer l'ouverture de service (4), plusieurs instruments chirurgicaux (10) destinés à un usage temporaire, et
- un support (12) comportant plusieurs éléments de fixation (14) configurés pour fixer les instruments chirurgicaux (10) au support (12) de manière détachable, le support (12) et les instruments chirurgicaux (10) formant un sous-ensemble (10.12) lorsque les instruments chirurgicaux (10) sont fixés au support (12) ;
le tube de stockage (2) étant configuré pour loger le sous-ensemble (10.12), de façon à stocker le sous-ensemble (10.12) à l'état stérile lorsque l'organe d'obturation (6) obture l'ouverture de service (4), et le sous-ensemble (10.12) étant conformé pour être extrait du tube de stockage (2) à travers l'ouverture de service (4) ; **caractérisé par**
le support (12) comprenant un manche (32) adapté pour être manipulé par un opérateur, le manche (32) ayant une portion d'attache configurée pour attacher sélectivement l'un des instruments chirurgicaux (10) de manière détachable, des éléments de fixation (14) étant formés par des rainures longitudinales (34) formées à la surface externe du manche (32), chaque rainure longitudinale (34) étant adaptée pour loger une partie d'un instrument chirurgical respectif (10) de façon à fixer ledit instrument chirurgical (10) par encliquetage élastique.

2. Ensemble d'instrumentation chirurgicale (1) selon la revendication 1, dans lequel les instruments chirurgicaux (10) sont sélectionnés dans le groupe constitué d'embouts de tournevis, de forets, de broches, d'enveloppes tubulaires contenant au moins une broche, de fraises à chambrer, de jauges et de réglets de mesure.

3. Ensemble d'instrumentation chirurgicale (1) selon l'une quelconque des revendications précédentes, dans lequel l'ouverture de service (4) est située à une extrémité du tube de stockage (2).

4. Ensemble d'instrumentation chirurgicale (1) selon la revendication 1, dans lequel le manche (32) présente une cavité centrale (38) adaptée pour loger au moins un instrument chirurgical (10).

5. Ensemble d'instrumentation chirurgicale (1) selon l'une quelconque des revendications précédentes, dans lequel le tube de stockage (2) a au moins une partie translucide, par exemple une partie transparente.

6. Ensemble d'instrumentation chirurgicale (1) selon l'une quelconque des revendications précédentes, dans lequel le tube de stockage (2) est composé d'un matériau sélectionné dans le groupe constitué d'un polyéthylène téréphtalate glycol (PETG), d'un polycarbonate (PC) et d'un polypropylène.

7. Ensemble d'instrumentation chirurgicale (1) selon l'une quelconque des revendications précédentes, dans lequel le tube de stockage (2) a une longueur (L2) comprise entre 70 mm et 300 mm, et dans lequel le tube de stockage (2) a une dimension transversale (W2), mesurée dans un plan perpendiculaire à la longueur (L2), comprise entre 20 mm et 100 mm.

8. Ensemble d'instrumentation chirurgicale (1) selon l'une quelconque des revendications précédentes, comprenant en outre :
- un tube de protection (42) configuré pour loger le tube de stockage (2), et
- un bouchon externe (44) configuré pour boucher le tube de protection (42) lorsque le tube de protection (42) loge le tube de stockage (2).

## Patentansprüche

1. Chirurgische Instrumentenanordnung (1), mindestens umfassend:
ein Lagerungsrohr (2), das eine Serviceöffnung (4) aufweist,
ein Verschlussorgan (6), das konfiguriert ist, um die Serviceöffnung (4) zu verschließen,
mehrere chirurgische Instrumente (10), die für eine vorübergehende Nutzung bestimmt sind, und
eine Halterung (12), die mehrere Befestigungselemente (14) beinhaltet, die konfiguriert sind, um die chirurgischen Instrumente (10) in abnehmbarer Weise an der Halterung (12) zu befestigen, wobei die Halterung (12) und die chirurgischen Instrumente (10) eine Unteranordnung (10.12) bilden, wenn die chirurgischen Instrumente (10) an der Halterung (12) befestigt sind;
wobei das Lagerungsrohr (2) konfiguriert ist, um die Unteranordnung (10.12) aufzunehmen, in der Art, um die Unteranordnung (10.12) im sterilen Zustand zu lagern, wenn das Verschlussorgan (6) die Serviceöffnung (4) verschließt, und die Unteranordnung (10.12) ausgeformt ist, um aus dem Lagerungsrohr (2) durch die Serviceöffnung (4) hindurch extrahiert zu werden; **gekennzeichnet durch**
die Halterung (12), die einen Griff (32) umfasst, der angepasst ist, um durch einen Operateur gehandhabt zu werden, wobei der Griff (32) einen Anbringungsabschnitt aufweist, der konfiguriert ist, um selektiv eines der chirurgischen Instrumente (10) in abnehmbarer Weise anzubringen, wobei die Befestigungselemente (14) durch Längsnuten (34) gebildet werden, die an der Außenfläche des Griffes (32) gebildet werden, wobei jede Längsnut (34) angepasst ist, um einen Teil eines entsprechenden chirurgischen Instruments (10) aufzunehmen, um das chirurgische Instrument (10) durch elastisches Einrasten zu befestigen.

2. Chirurgische Instrumentenanordnung (1) nach Anspruch 1, wobei die chirurgischen Instrumente (10) aus der Gruppe ausgewählt sind, die aus Einsätzen von Schraubendrehern, von Bohrern, von Stiften, von rohrförmigen Hüllen, die mindestens einen Stift enthalten, von Senkfräsern, von Messgeräten und Messskalen besteht.

3. Chirurgische Instrumentenanordnung (1) nach einem der vorstehenden Ansprüche, wobei sich die Serviceöffnung (4) an einem Ende des Lagerungsrohres (2) befindet.

4. Chirurgische Instrumentenanordnung (1) nach Anspruch 1, wobei der Griff (32) einen zentralen Hohlraum (38) aufweist, der angepasst ist, um mindestens ein chirurgisches Instrument (10) aufzunehmen.

5. Chirurgische Instrumentenanordnung (1) nach einem der vorstehenden Ansprüche, wobei das Lagerungsrohr (2) mindestens ein durchscheinendes Teil, beispielsweise ein durchsichtiges Teil aufweist.

6. Chirurgische Instrumentenanordnung (1) nach einem der vorstehenden Ansprüche, wobei sich das Lagerungsrohr (2) aus einem Material zusammensetzt, das aus der Gruppe ausgewählt wird, die aus einem Polyethylenterephtalatglykol (PETG), einem Polycarbonat (PC), und einem Polypropylen gebildet wird.

7. Chirurgische Instrumentenanordnung (1) nach einem der vorstehenden Ansprüche, wobei das Lagerungsrohr (2) eine Länge (L2) aufweist, die zwischen 70 mm und 300 mm enthalten ist, und wobei das Lagerungsrohr (2) eine Querabmessung (W2) aufweist, die in einer Ebene senkrecht zur Länge (L2) gemessen, zwischen 20 mm und 100 mm enthalten ist.

8. Chirurgische Instrumentenanordnung (1) nach einem der vorstehenden Ansprüche, weiter Folgendes umfassend:
- ein Schutzrohr (42), das konfiguriert ist, um das Lagerungsrohr (2) aufzunehmen, und
- einen äußeren Stopfen (44), der konfiguriert ist, um das Schutzrohr (42) zuzustopfen, wenn das Schutzrohr (42) das Lagerungsrohr (2) aufnimmt.

## Claims

1. A surgical instrument set (1) comprising at least:
- one storage tube (2) having a service opening (4),
- one sealing member (6) configured to seal the service opening (4),
- several surgical instruments (10) intended for a temporary use, and
- one support (12) including several fastening elements (14) configured to fasten the surgical instruments (10) to the support (12) in a detachable manner, the support (12) and the surgical instruments (10) forming a subset (10.12) when the surgical instruments (10) are fastened to the support (12);
the storage tube (2) being configured to house the subset (10.12), so as to store the subset (10.12) in the sterile state when the sealing member (6) seals the service opening (4), and the subset (10.12) being shaped to be extracted from the storage tube (2) through the service opening (4); **characterized by**
the support (12) comprising a handle (32) suitable to be manipulated by an operator, the handle (32) having an attachment portion configured to selectively attach one of the surgical instruments (10) in a detachable manner, fastening elements (14) being formed by longitudinal grooves (34) formed on the outer surface of the handle (32), each longitudinal groove (34) being suitable to house a portion of a respective surgical instrument (10) so as to fasten said surgical instrument (10) by elastic snapfit.

2. The surgical instrument set (1) according to claim 1, wherein the surgical instruments (10) are selected from the group consisting of screwdriver bits, drills, pins, tubular casings containing at least one pin, countersinks, gauges and measurement rulers.

3. The surgical instrument set (1) according to any one of the preceding claims, wherein the service opening (4) is located at an end of the storage tube (2).

4. The surgical instrument assembly (1) according to claim 1, wherein the handle (32) has a central cavity (38) suitable to house at least one surgical instrument (10).

5. The surgical instrument set (1) according to any one of the preceding claims, wherein the storage tube (2) has at least one translucent portion, for example a transparent portion.

6. The surgical instrument set (1) according to any one of the preceding claims, wherein the storage tube (2) is composed of a material selected from the group consisting of a polyethylene terephthalate glycol (PETG), a polycarbonate (PC) and a polypropylene.

7. The surgical instrument set (1) according to any one of the preceding claims, wherein the storage tube (2) has a length (L2) comprised between 70 mm and 300 mm, and wherein the storage tube (2) has a transverse dimension (W2), measured in a plane perpendicular to the length (L2), comprised between 20 mm and 100 mm.

8. The surgical instrument set (1) according to any one of the preceding claims, further comprising:
- a protection tube (42) configured to house the storage tube (2), and
- an outer plug (44) configured to plug the protection tube (42) when the protection tube (42) houses the storage tube (2).
